# EUROPEAN PATENT APPLICATION

(11) **EP 2 944 346 A1**
(43) Date of publication of application: **18.11.2015**
(21) Application number: 13870532.2
(22) Date of filing: 26.12.2013
(51) Int. Cl.: A61M 25/06, A61M 5/158

(54) **SAFE VENOUS INDWELLING NEEDLE**

(30) Priority: 11.01.2013 CN 201310011208
(71) Applicant: Sunwell Biotech Co., Ltd., Jiangsu (CN)
(72) Inventor: LI, Zhi-Yun, Nantong Jiangsu (CN)
(74) Representative: Baldwin, Mark
(86) International application number: PCT/CN2013/090485
(87) International publication number: WO 2014/108027

(57) **Abstract**

The present invention is related to a safe vein detained needle, comprises a detained needle seat fixedly provided with a detained trocar, a protective seat, a puncture needle seat fixedly provided with a puncture needle, a withdrawing part, and a pushing seat. Before a puncturing process, the pushing seat is pushed forward, such that the withdrawing part is coupled to the puncture needle seat. After the puncturing process is completed, then, the pushing seat is pushed backward, such that the puncture needle seat is drawn backward along with the coupled withdrawing part, so as to retrieve the puncture needle on the puncture needle seat into the protective seat. Thereby, the puncture needle is retrieved into the protective seat after the puncturing process is completed, so as to avoid the risk of accidental pricking wound to medical personnel by the used puncture needle.

## Description

### FIELD OF THE INVENTION

The present invention is related to a safe vein detained needle, particularly to a safe vein detained needle for preventing needle pricking, capable of avoiding the risk of accidental pricking would to medical personnel caused by a used puncture needle.

### BACKGROUND

For the prevention of pain caused by puncturing for injection several times, a vein detained needle is generally utilized for the patient, who requires intravenous injection several times, to facilitate injection, so as to avoid the pain caused by injection several times.

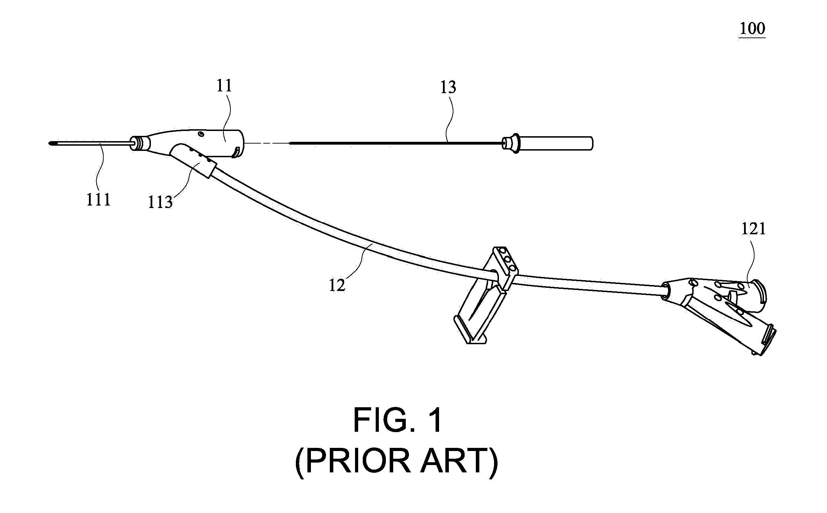

Referring to Fig. 1, there is shown an exploded perspective view of the structure of an existed vein detained needle. As illustrated in the figures, the vein detained needle 100 comprises a detained needle seat 11 and a puncture needle 13.

Wherein, the detained needle seat 11 is provided at the front end thereof with a detained trocar 111, while communicated on the side wall thereof with a shunt tube 113, the shunt tube 113 being coupled to a transfusion tube 12. The transfusion tube 12, used as a passage for medicament injection or blood flow, is provided with a joint portion 121 used to fit with a pipe, conduit or vessel capable of containing medicament or blood. Further, the needle body of the puncture needle 13 may be embeddedly provided inside the detained needle seat 11, while a part of the needle body is exposed to the outside of the front end of the detained trocar 111. Furthermore, before the vein detained needle 100 is not used yet, a needle sheath may be used to cover the front end of the detained needle seat 11, so as to cover the detained trocar 111 and the puncture needle 13 passing therethrough.

When the vein detained needle 100 is used clinically, the needle sheath may be removed by medical personnel, in such a way that the detained trocar 111 at the front end of the detained needle seat 11 and the puncture needle 13 passing therethrough are exposed outside. The puncture needle 13 is allowed for leading the detained trocar 111 to enter the vein of human body. When the puncture of vein is confirmed after blood return is found, the puncture needle 13 is withdrawn, detaining the detained trocar 111 in the vein only. Afterwards, medicament may be also infused into human body through the transfusion tube 12, shunt tube 113 and the detained trocar 111.

Formerly, in the conventional vein detained needle 100, the puncture needle 13 is exposed outside completely when the puncture needle 13 is withdrawn. This puncture needle 13 adhered with blood of the patient is probable to wound accidentally and infect medical personnel, as well as troublesome for medical waste disposal. Thereby, there is secret worry existed in medical protection.

Here, the present invention provides a vein detained needle capable of preventing needle pricking, in which may reducing the risk of accidental pricking would infection to medical personnel when the medical personnel retrieves the puncture needle of the vein detained needle, which is the object to be achieved by the present invention desirably.

### SUMMARY OF THE INVENTION

It is one object of the present invention to provide a safe vein detained needle, comprising a detained needle seat fixedly provided with a detained trocar, a protective seat, a puncture needle seat fixedly provided with a puncture needle, and a pushing seat embeddedly provided with a withdrawing part, the pushing seat allowed for sliding back and forth on the protective seat. Before a puncturing process, the pushing seat is pushed forward, such that the withdrawing part is coupled to the puncture needle seat, and after the withdrawing part being coupled to the puncture needle seat, the detained trocar is led to puncture the vein of human body by means of the puncture needle on the puncture needle seat. After the puncturing process is finished, the pushing seat is pushed backward, such that the puncture needle seat is drawn backward along with the coupled withdrawing part, so as to retrieve the puncture needle on the puncture needle seat into the protective seat, thus avoiding the risk of accidental pricking wound to medical personnel by the used puncture needle.

It is another object of the present invention to provide a safe vein detained needle, wherein a needle head accommodating portion is provided within the protective seat, and at least one groove is formed on an inner side wall at the front end of the needle head accommodating portion. When the withdrawing part is coupled to the puncture needle seat, leverage is generated between the withdrawing part and the puncture needle seat, so as to force the puncture needle to enter the groove of the needle head accommodating portion obliquely when the puncture needle being withdrawn to the protective seat, whereby the probability of allowing the puncture needle passing out from the center of the protective seat again is reduced.

It is a further object of the present invention to provide a safe vein detained needle, wherein a spring assembly is provided at the rear end of the protective seat. The pushing part pushes the pushing seat backward so as to press the spring assembly to be compressed. Further, after the pushing part is released, the puncture needle seat is pushed forward by the resilient pushing force of the spring assembly, such that the needle head of the puncture needle on the puncture needle seat may be stably butted forward into the groove of the needle head accommodating portion deeply, further enhancing safety of retrieving puncture needle.

For achieving aforementioned objects, the present invention provides a safe vein detained needle, comprising a detained needle seat provided at the center thereof with a through bore, and on the side wall thereof with a shunt tube communicated with the through bore, wherein the front end of the through bore is fixedly with a detained trocar exposed to the outside of the detained needle seat; a protective seat provided at the front end thereof with a needle head accommodating portion, and at the rear end thereof with a curved part, wherein the protective seat being joined to the through bore of the detained needle seat; a puncture needle seat insertedly fixed at the front end thereof with a puncture needle, and provided at the rear end thereof with a first coupling portion, wherein when the puncture needle seat is accommodated in the needle head accommodating portion, the puncture needle passing through a front hole at the center of body of the protective seat, the through bore and the detained trocar of the detained needle seat, so as to be exposed outside of the front end of the detained trocar; a withdrawing part provided at the front end thereof with a second coupling portion; and a pushing seat presented as a hollow tube, the tube being provided on the peripheral thereof with a pushing part and embeddedly fixed at the center thereof with the withdrawing part, the curved part of the protective seat being passingly provided between the peripheral of tube and the center of tube, the pushing seat sliding back and forth on the curved part of the protective seat under pushing action of the pushing part, wherein the second coupling portion of the withdrawing part and the first coupling portion of the puncture needle seat being coupled together, when the pushing part is pushed forward, the puncture needle seat coupled to the withdrawing part being drawn backward along therewith, when the pushing part is pushed backward, such that the puncture needle is returned into the needle head accommodating portion.

In one embodiment of the present invention, the first coupling portion is presented as a recessed structure, and the second coupling portion is presented as a raised structure, the first coupling portion being provided on an inner side wall thereof with a projection and a thin-sided surface, while on another inner side wall thereof with a thick-sided surface, wherein when the second coupling portion is coupled to the first coupling portion, leverage being generated between the puncture needle seat and the withdrawing part, so as to force the puncture needle to oblique when the puncture needle is withdrawn backward to the needle head accommodating portion.

In one embodiment of the present invention, the pushing seat is provided at the center of tube thereof with a round hole, and between the peripheral of tube and the round hole thereof with an arc-shaped aperture, the withdrawing part being embeddedly fixed in the round hole, while the curved part of the protective seat being passingly provided in the arc-shaped aperture.

In one embodiment of the present invention, wherein at least one groove is formed on an inner side wall at the front end of the needle head accommodating portion, the needle head of the puncture needle entering the groove obliquely when the puncture needle is withdrawn to the needle head accommodating portion.

In one embodiment of the present invention, wherein a ramp is formed between the groove and the front hole, the needle head of the puncture needle entering the groove obliquely along the ramp when the puncture needle is withdrawn to the needle head accommodating portion through the front hole.

In one embodiment of the present invention, further comprises a spring assembly, the spring assembly being fixedly provided at the rear end of the protective seat, wherein the pushing part pushing the pushing seat is moved backward so as to press the spring assembly to be compressed, while the pushing seat, the withdrawing part, and the puncture needle seat being pushed forward by resilient pushing force of the spring assembly after the pushing part is released, such that the needle head of the puncture needle on the puncture needle seat is butted forward into the groove deeply.

In one embodiment of the present invention, wherein the spring assembly comprises a pressing part, a spring part, and a receiving seat, the pressing part and the spring part being accommodated in the receiving seat, the spring assembly being assembled to a rear accommodating opening of the protective seat by means of the receiving seat.

In one embodiment of the present invention, wherein the pushing part is provided on the top thereof with a finger pushing portion presented as depressed mode, while at two sides thereof with a pair of wing portions having anti-slip pattern.

In one embodiment of the present invention, wherein the detained needle seat further comprises an isolating plug therein, the isolating plug being provided at the rear end of junction between the through bore and the shunt tube, the puncture needle passing through the through bore by puncturing the isolating plug.

In one embodiment of the present invention, wherein the protective seat is provided on the periphery at the front end thereof with a U-shaped stopping portion, a part of tube of the pushing seat is provided with a U-shaped gap, correspondingly, and the curved part of the protective seat is provided at the rear end thereon with a marking curve, wherein the second coupling portion of the withdrawing part being coupled to the first coupling portion of the puncture needle seat when the pushing part is pushed forward such that the gap of the pushing seat is adjoined to the stopping portion, alternatively, the needle head of the puncture needle being returned to be retrieved into the needle head accommodating portion when the pushing part is pushed backward such that the gap of the pushing seat is moved to the top of the marking curve.

In the following, the present invention will be described in detail in combination with attached drawings and embodiments, but not be limited thereto.

### BRIEF DESCRIPTION OF DRAWINGS

Fig. 1 is an exploded perspective view of the structure of an existed vein detained needle;
Fig. 2 is an exploded perspective view of the structure a safe vein detained needle of the present invention;
Fig. 3 is an exploded sectional view of the structure of the safe vein detained needle of the present invention;
Fig. 4 is a an assembled perspective view of the structure of the safe vein detained needle of the present invention;
Fig. 5 is an assembled sectional view of the structure of the safe vein detained needle of the present invention;
Fig. 6 is an enlarged perspective view of partial structure of a protective seat of the present invention;
Fig. 7A to 7E are a structural perspective view, a top view, a side view, a structural sectional view along A-A', a structural sectional view along B-B', respectively, of a pushing seat of the present invention;
Fig. 8 is an enlarged view of partial structure of Fig. 5 of the present invention;
Fig. 9 is an exploded sectional view of the structure of the safe vein detained needle of the present invention showing the pushing seat being pushed forward;
Fig. 10 is an enlarged view of partial structure of Fig. 9 of the present invention;
Fig. 11 is an exploded sectional view of the structure of the safe vein detained needle of the present invention showing the pushing seat being pushed backward;
Fig. 12 is an enlarged view of partial structure of Fig. 11 of the present invention.

**LIST OF REFERENCE SYMBOLS**

| | | | |
|---|---|---|---|
| 100 | vein detained needle | 11 | detained needle seat |
| 111 | detained trocar | 113 | shunt tube |
| 12 | transfusion tube | 121 | joint portion |
| 13 | puncture needle | 200 | vein detained needle |
| 21 | detained needle seat | 211 | detained trocar |
| 212 | through bore | 2120 | first fastener |
| 213 | shunt tube | 214 | isolating plug |
| 215 | transfusion tube | 22 | protective seat |
| 221 | needle head accommodating portion | 2211 | groove |
| 2213 | ramp | 222 | curved part |
| 2221 | marking curve | 223 | front hole |
| 2230 | second fastener | 224 | stopping portion |
| 225 | rear accommodating opening | 23 | puncture needle seat |
| 231 | puncture needle | 232 | first coupling portion |
| 2321 | protrusion | 2323 | thin-sided surface |
| 2325 | thick-sided surface | 24 | pushing seat |
| 241 | pushing part | 2411 | wing portions |
| 2413 | finger pushing portion | 243 | round hole |
| 2431 | first embedding portion | 244 | gap |
| 245 | arc-shaped aperture | 25 | withdrawing part |
| 251 | second embedding portion | 252 | second coupling portion |
| 26 | spring assembly | 261 | pressing part |
| 262 | spring part | 263 | receiving seat |

### DETAILED DESCRIPTION

Thereinafter, the structural principle and operating principle of the present invention will be described specifically in accordance with attached drawings.

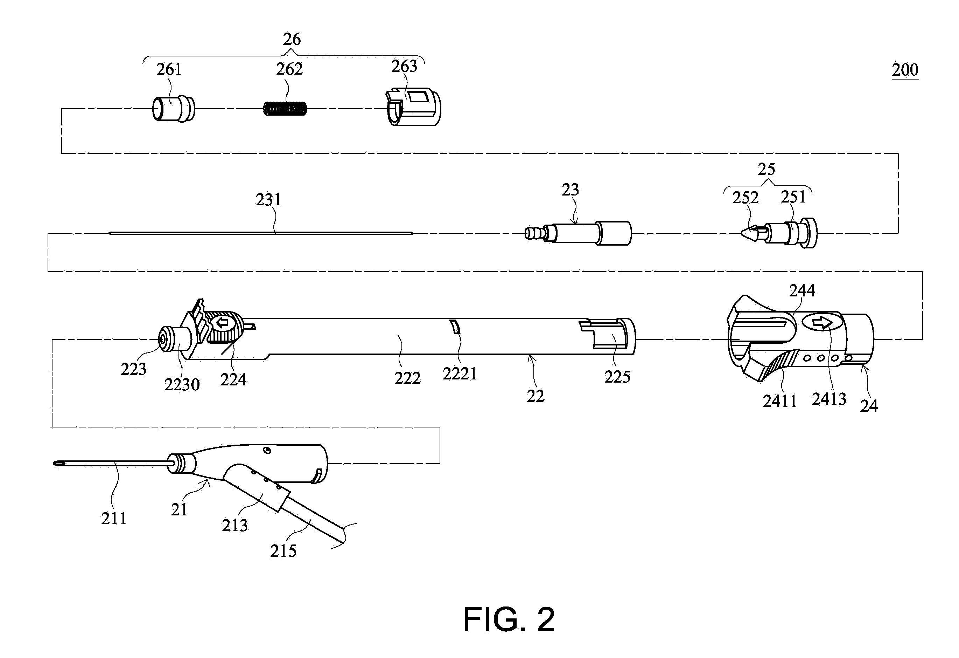

Referring to Figs. 2, 3, 4 and 5, there are shown an exploded perspective view, exploded sectional view, assembled perspective view and assembled sectional view, respectively, of the structure of a safe vein detained needle according to one preferred embodiment of the present invention. As illustrated in the figures, a vein detained needle 200 comprises a detained needle seat 21, a protective seat 22, a puncture needle seat 23, a pushing seat 24 and a withdrawing part 25.

Wherein the detained needle seat 21 is provided at the center thereof with a through bore 212, and at the side wall thereof with a shunt tube 213 communicated to the through bore 212. The shunt tube 213 is couplingly provided with a transfusion tube 215, used as a passage for medicament injection or blood flow. Furthermore, at the front end of the through bore 212, there is fixedly provided with a detained trocar 211 exposed outside of the detained needle seat 21; while at the rear end of this through bore 212, there is an opening.

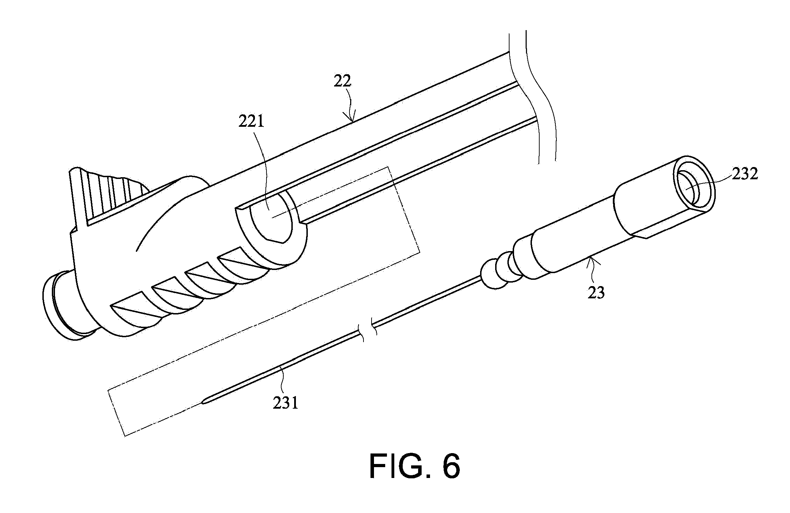

Further, referring to Fig. 6 together, the protective seat 22 is provided at the front end thereof with a needle head accommodating portion 221 presented as a hollow tube, and formed at the center of the front end thereof with a front hole 223, while presented as a curved part 222 at the rear end thereof. In one embodiment of the present invention, the front end of the protective seat 22 may be also joined in the opening at the rear end of the detained needle seat 21. In a further embodiment of the present invention, a first fastener 2120 is further provided at the inner edge of the opening of the rear end of the detained needle seat 21, while a second fastener 2230 is further provided on the periphery of the front hole 223 of the protective seat 22. The detained needle seat 21 and the protective seat 22 are joined together stably by means of fastening between the first fastener 2120 and the second fastener 2230 (for example, projection and recess corresponding to each other).

At the front end of the puncture needle seat 23, there is insertedly fixed with a puncture needle 231. When the puncture needle seat 23 is provided in the needle head accommodating portion 221 of the protective seat 22, the puncture needle 231 may be exposed to the outside of the front end of the detained trocar 211 passing through the front hole 223 of the protective seat 22, the through bore 212 of the detained needle seat 21, and the detained trocar 211 in turn. Furthermore, the detained needle seat 21 further comprises an isolating plug 214 therein, the isolating plug being provided at the rear end of junction between the through bore 212 and the shunt tube 213. The puncture needle 231 is allowed for passing through the through bore 212 by puncturing the isolating plug 214. The isolating plug 214 is used for gripping the puncture needle 231, so as to avoid the sliding of the puncture needle 231 generated when the detained trocar 211 is led by the puncture needle 231 to puncture the vein.

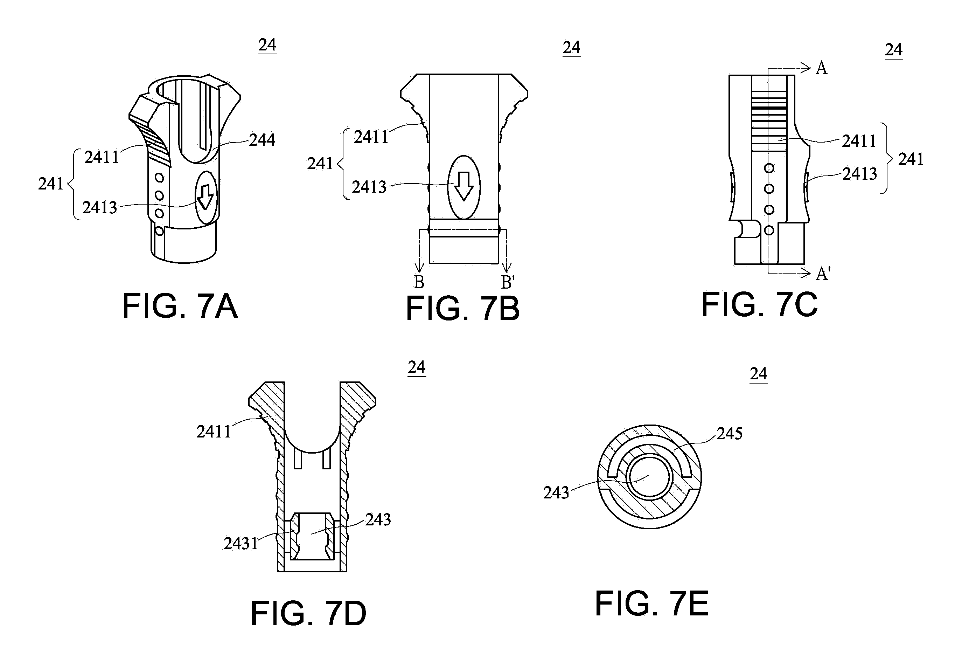

Further, referring to Figs. 7A to 7E together, the pushing seat 24 is presented as a hollow tube, the periphery of the tube is provided with a pushing part 241, the center of the tube is provided with a round hole 243, while between the periphery of tube and the central round hole 243 is provided with an arc-shaped aperture 245. In the present invention, the withdrawing part 25 is embeddedly fixed in the round hole 243, while the curved part 222 of the protective seat 22 is passingly provided in the arc-shaped aperture 245. The pushing seat 24 is allowed to slide back and forth on the curved part 222 of the protective seat 22 under the pushing action of the pushing part 241, as well as the withdrawing part 25 embeddedly fixed in the pushing seat 24 is also allowed to operate correspondingly along with the pushing seat 24. In one embodiment of the present invention, a first embedding portion 2431 is provided within the round hole 243 of the pushing seat 24, while a second embedding portion 251 is surroundingly provided on the periphery of the withdrawing part 25. The withdrawing part 25 is embeddedly fixed within the first embedding portion 2431 via the second embedding portion 251.

Further, a first coupling portion 232 is provided at the rear end of the puncture needle seat 23, while a second coupling portion 252 is provided at the front end of the withdrawing part 25, correspondingly. When medical personnel is desired to puncture with respect to the patient, the pushing part 241 pushing the pushing seat 24 is moved forward, such that the withdrawing part 25 may be driven to move forward by the pushing seat 24, leading the second coupling portion 252 of the withdrawing part 25 coupling to the first coupling portion 232 of the puncture needle seat 23. After the withdrawing part 25 is coupled to the puncture needle seat 23, the puncture needle 231 on the puncture needle seat 23 is used by medical personnel to lead the detained trocar 211 to puncture the vein of human body. Then, after the puncture is confirmed by medical personnel, the pushing part 241 pushing the pushing seat 24 is moved backward, such that the withdrawing part 25 and the puncture needle seat 23 coupled thereto may be driven backward by the pushing seat 24, leading the puncture needle 231 on the puncture needle seat 23 to be returned into the needle head accommodating portion 221 of the protective seat 22. Thereby, the object of retrieving puncture needle 231 safely may be achieved. Furthermore, the coupling manner between the first coupling portion 232 and the second coupling portion 252 will be detailed later.

Further, the protective seat 22 is provided on the periphery at the front end thereof with a U-shaped stopping portion 224, while a part of tube of the pushing seat 24 is provided with a U-shaped gap 244, correspondingly. When the pushing part 241 is pushed forward such that the gap 244 of the pushing seat 24 is adjoined to the stopping portion 224, the second coupling portion 252 of the withdrawing part 25 may be coupled to the first coupling portion 232 of the puncture needle seat 23. In addition, the rear curved part 222 of the protective seat 22 is provided thereon with a marking curve 2221. When the pushing part 241 is pushed backward such that the gap 244 of the pushing seat 24 is moved to the top of the marking curve 2221, the needle head of the puncture needle 231 on the puncture needle seat 23 may be then returned to be retrieved into the needle head accommodating portion 22. In addition, the pushing part 241 is provided at two sides thereof with a pair of wing portions 2411 having anti-slip pattern, while on the top thereof with a finger pushing portion 2413 presented as depressed shape. It is also allowed for medical personnel to select the wing portion 2411 of the pushing part 241 to push the pushing seat 24 forward, or the finger pushing portion 2413 of the pushing part 241 to push the pushing seat 24 backward.

Furthermore, the vein detained needle 200 of the present invention further comprises a spring assembly 26. The spring assembly 26 comprises a pressing part 261, a spring part 262, and a receiving seat 263. Wherein the pressing part 261 and the spring part 262 are accommodated in the receiving seat 263, while the spring assembly 26 is assembled to a rear accommodating opening 225 of the protective seat 22 by means of the receiving seat 263. Further, the inner side wall at the front end of the needle head accommodating portion 221 is formed with at least one groove 2211, with a ramp 2213 being formed between each groove 221 and the front hole 223, respectively.

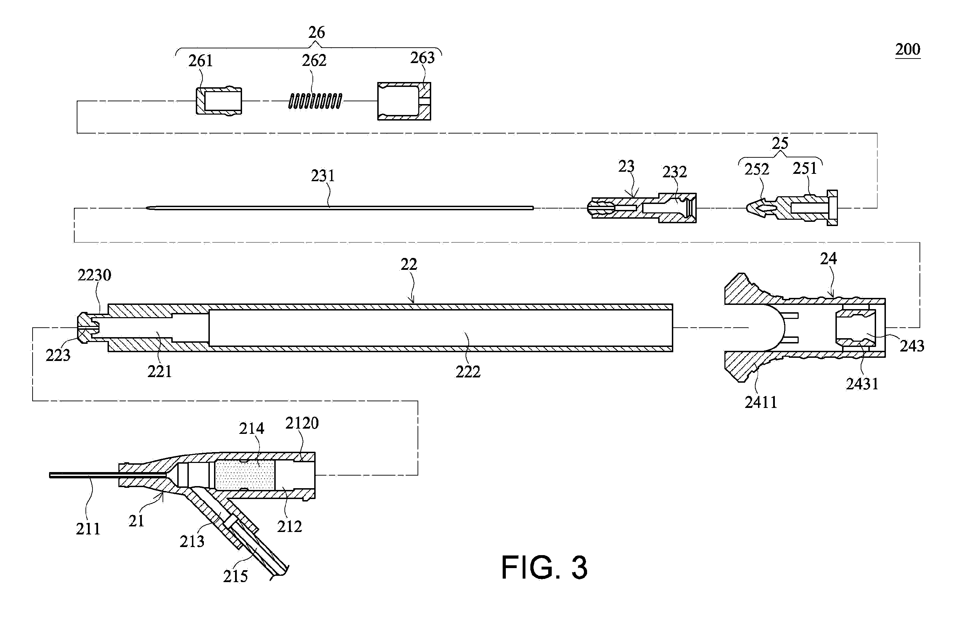

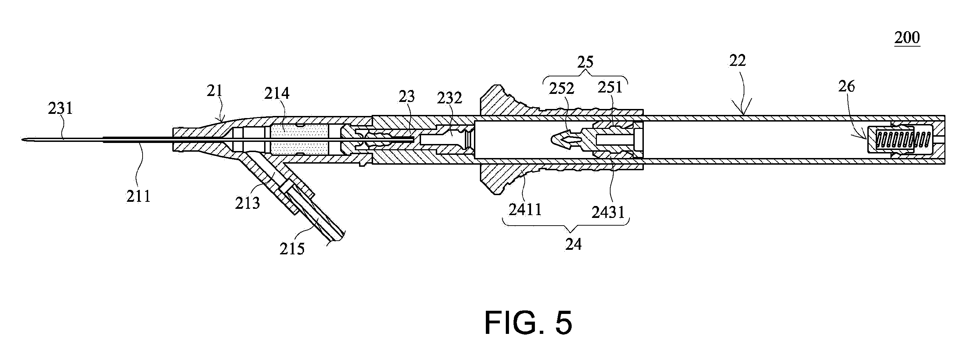

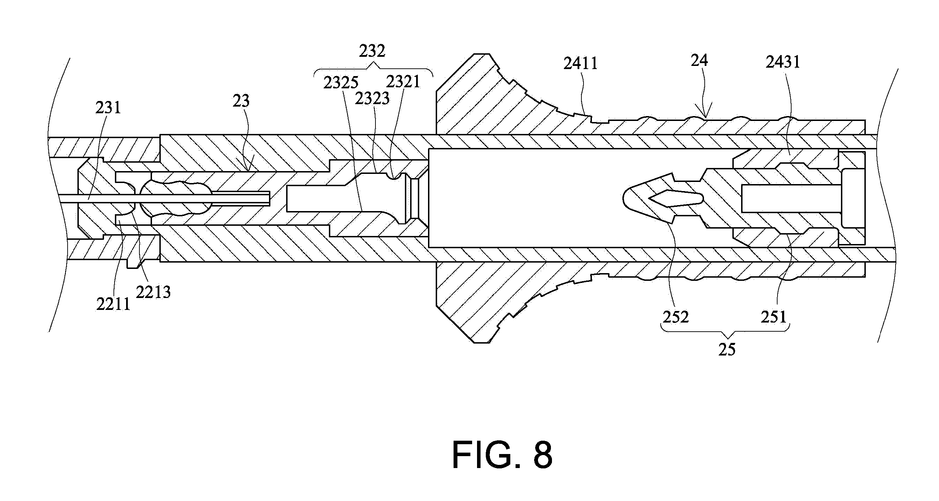

Referring to Fig. 8, which is an enlarged view of partial structure of Fig. 5, one embodiment of the present invention is illustrated. In this case, the first coupling portion 232 of the puncture needle seat 23 is presented as a recessed structure, while the second coupling portion 252 of the withdrawing part 25 is presented as a resilient raised structure. On an inner side wall of the first coupling portion 232, a protrusion 2321 and a thin-sided surface 2323 are provided, while on another inner side wall, a thick-sided surface 2325 is provided. When the second coupling portion 252 is coupled to the first coupling portion 232, the second coupling portion 252 is pressed by the protrusion 2321 within the first coupling portion 232 toward the thick-sided surface 2325, such that leverage is generated between the puncture needle seat 23 and the withdrawing part 25. Afterwards, when the puncture needle 231 on the puncture needle seat 23 is withdrawn backward out of the front hole 223 (as illustrated in Figs. 2 and 3), the puncture needle seat 23 and the puncture needle 231 fixedly provided thereon may be obliqued due to leverage, so as to avoid the puncture needle 231 passing out from the central front hole 223 of the protective seat 22 (as illustrated in Figs.2 and 3) again, and thus accidental pricking wound to medical personnel. Furthermore, the interior of the second coupling portion 252 described in the present invention is presented as a hollow structure, in such a way that the second coupling portion 252 is provided with better resilience, facilitating the second coupling portion 252 to couple to the first coupling portion 232.

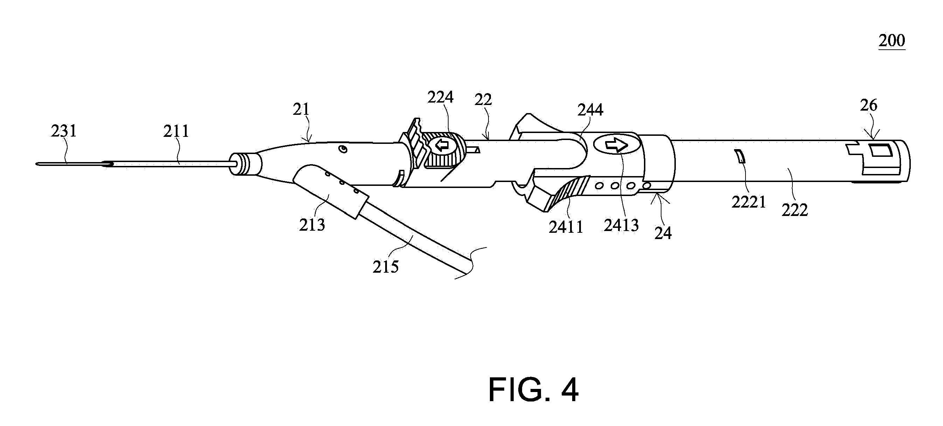

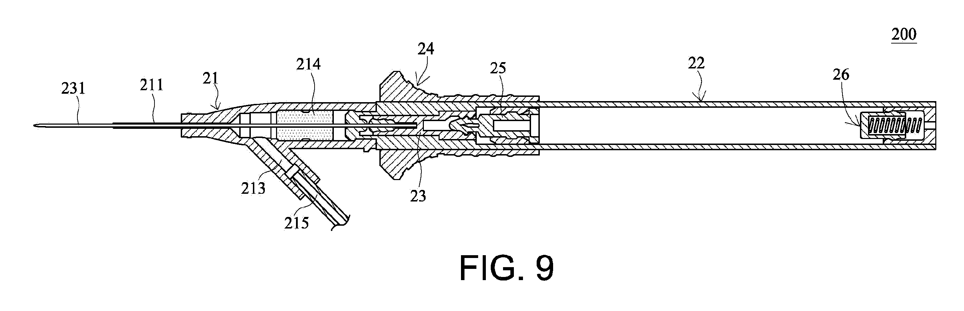

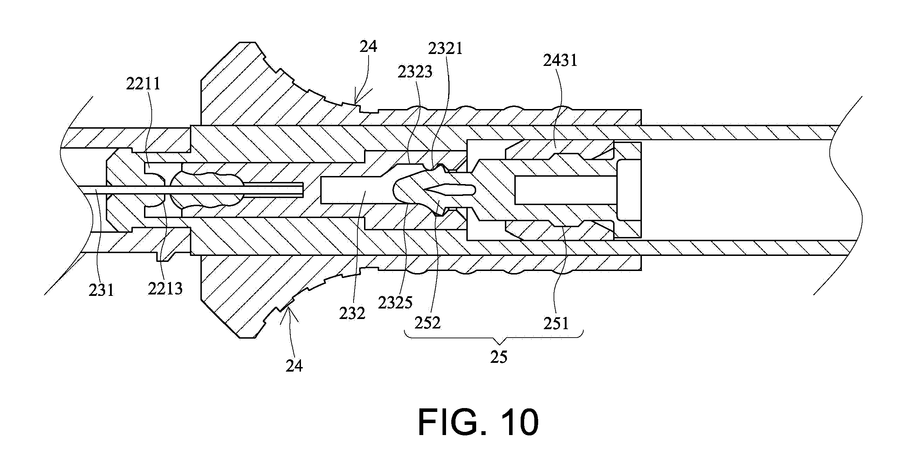

Subsequently, the detailed operation of the puncture needle 231 in the puncturing process and retrieving process is described as follows. Firstly, referring to Figs. 9 and 10, when medical personnel is desired to perform a puncturing process with respect to the patient, the pushing part 241 of the pushing seat 24 may be pushed forward to the position of the stopping portion 224 (as illustrated in Fig. 4), such that the second coupling portion 252 of the withdrawing part 25 being coupled to the first coupling portion 232 of the puncture needle seat 23. At this time, the puncture needle 231 on the puncture needle seat 23 is used by medical personnel to lead the detained trocar 211 to puncture the vein of human body. Moreover, when the second coupling portion 252 of the withdrawing part 25 is coupled to the first coupling portion 232 of the puncture needle seat 23, the second coupling portion 252 is pressed by the protrusion 2321 within the first coupling portion 232 toward the thick-sided surface 2325, such that leverage is generated between the puncture needle seat 23 and the withdrawing part 25.

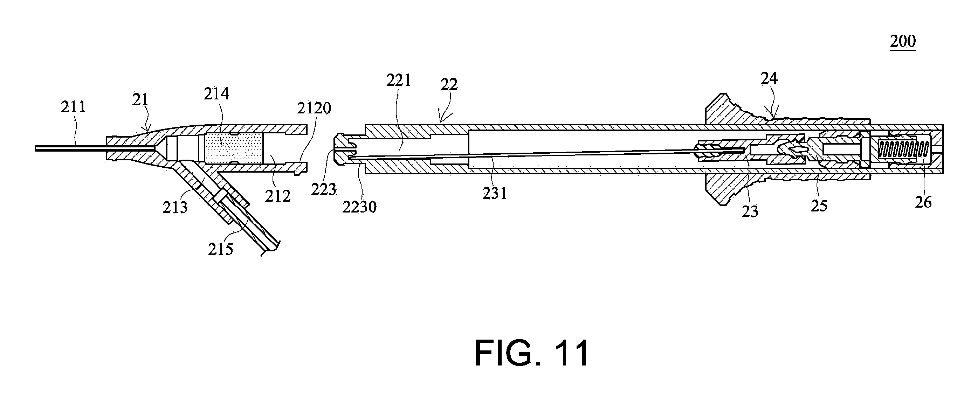

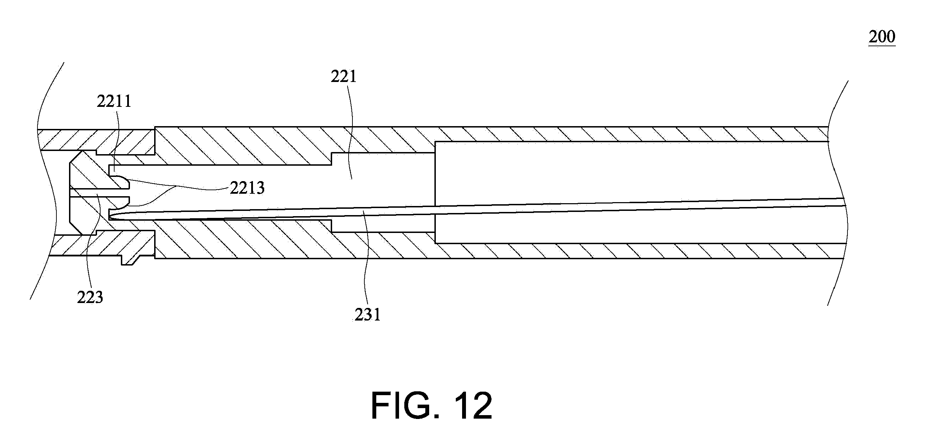

Referring to Figs. 11 and 12 successively, after venipuncture is confirmed by medical personnel, the retrieving process is performed on the puncture needle 231, in which the pushing part 241 pushing the pushing seat 24 is moved backward, and the puncture needle seat 23 is then drawn backward along with the withdrawing part 25 coupled together. When the pushing part 241 pushing the pushing seat 24 is drawn backward to the position of the marking curve 2221 (as illustrated in Fig. 4), the puncture needle 231 may be separated out of the front hole 223. Thus, the puncture needle 231 may be obliqued to enter the groove 2211 due to leverage generated between the first coupling portion 232 and the second coupling portion 252. At this time, the second coupling portion 252 may be leaned against the thin-sided surface 2323. Further, when the pushing part 241 pushing the pushing seat 24 is drawn backward to the position of the marking curve 2221 (as illustrated in Fig. 4), the bottom of the withdrawing part 25 is pressed against, and then compress the spring assembly 26. Afterwards, the pushing part 241 is released, and the puncture needle 231 may be then moved forward obliquely by means of leverage generated between the first coupling portion 232 and the second coupling portion 252 together with resiliently pushing force of the spring assembly 26. In cooperation with the design of ramp 2213, moreover, the needle head of the puncture needle 231 may be obliqued to enter the groove 2211 along the ramp 2213. In this way, the needle head of the puncture needle 231 may be moved forward to be butted into the groove 2211 deeply under the resiliently pushing force of the spring assembly 26. The needle head of the puncture needle 231 may be then retrieved into the protective seat 22 safely without fear, so as to avoid the risk of accidental pricking wound to medical personnel by a used puncture needle 231. Afterwards, when the needle head of the puncture needle 231 is returned into the protective seat 22 securely, medical personnel is allowed to remove the protective seat 22 out of the opening at the rear end of the detained needle seat 21. Thereby, the retrieving process of puncture needle 231 is completed.

Naturally, there are still various embodiments for the present invention. It should be understood that various changes and alterations could be made to the present invention by those skilled in the art without departing from the spirit and scope of the invention, and included within the scope of the appended claims.

## Claims

1. A safe vein detained needle, comprises:
a detained needle seat provided at the center thereof with a through bore, and on the side wall thereof with a shunt tube communicated with said through bore, wherein the front end of said through bore is fixedly with a detained trocar exposed to the outside of said detained needle seat;
a protective seat provided at the front end thereof with a needle head accommodating portion, and at the rear end thereof with a curved part, wherein said protective seat being joined to said through bore of said detained needle seat;
a puncture needle seat insertedly fixed at the front end thereof with a puncture needle, and provided at the rear end thereof with a first coupling portion, wherein when said puncture needle seat is accommodated in said needle head accommodating portion, said puncture needle passing through a front hole at the center of body of said protective seat, said through bore and said detained trocar of said detained needle seat so as to be exposed outside of the front end of said detained trocar;
a withdrawing part provided at the front end thereof with a second coupling portion; and
a pushing seat presented as a hollow tube, said tube being provided on the peripheral thereof with a pushing part and embeddedly fixed at the center thereof with said withdrawing part, said curved part of said protective seat being passingly provided between the peripheral of tube and the center of tube, said pushing seat sliding back and forth on said curved part of said protective seat under pushing action of said pushing part, wherein said second coupling portion of said withdrawing part and said first coupling portion of said puncture needle seat being coupled together when said pushing part is pushed forward, said puncture needle seat coupled to said withdrawing part being drawn backward along therewith when said pushing part is pushed backward, such that said puncture needle is returned into said needle head accommodating portion.

2. The safe vein detained needle according to Claim 1, wherein said first coupling portion is presented as a recessed structure, and said second coupling portion is presented as a raised structure, said first coupling portion being provided on an inner side wall thereof with a projection and a thin-sided surface, while on another inner side wall thereof with a thick-sided surface, wherein when said second coupling portion is coupled to said first coupling portion, leverage being generated between said puncture needle seat and said withdrawing part, so as to force said puncture needle to oblique when said puncture needle is withdrawn backward to said needle head accommodating portion.

3. The safe vein detained needle according to Claim 1, wherein said pushing seat is provided at the center of tube thereof with a round hole, and between the peripheral of tube and said round hole thereof with an arc-shaped aperture, said withdrawing part being embeddedly fixed in said round hole, while said curved part of said protective seat being passingly provided in said arc-shaped aperture.

4. The safe vein detained needle according to Claim 2, wherein at least one groove is formed on an inner side wall at the front end of said needle head accommodating portion, said needle head of said puncture needle entering said groove obliquely when said puncture needle is withdrawn to said needle head accommodating portion.

5. The safe vein detained needle according to Claim 4,wherein a ramp is formed between said groove and said front hole, said needle head of said puncture needle entering said groove obliquely along said ramp when said puncture needle is withdrawn to said needle head accommodating portion through said front hole.

6. The safe vein detained needle according to Claim 4, further comprises a spring assembly, said spring assembly being fixedly provided at the rear end of said protective seat, wherein said pushing part pushing said pushing seat is moved backward so as to press said spring assembly to be compressed, while said pushing seat, said withdrawing part, and said puncture needle seat being pushed forward by resilient pushing force of said spring assembly after said pushing part is released, such that said needle head of said puncture needle on said puncture needle seat is butted forward into said groove deeply.

7. The safe vein detained needle according to Claim 6, wherein said spring assembly comprises a pressing part, a spring part, and a receiving seat, said pressing part and said spring part being accommodated in said receiving seat, said spring assembly being assembled to a rear accommodating opening of said protective seat by means of said receiving seat.

8. The safe vein detained needle according to Claim 1, wherein said pushing part is provided on the top thereof with a finger pushing portion presented as depressed shape, while at two sides thereof with a pair of wing portions having anti-slip pattern.

9. The safe vein detained needle according to Claim 1, wherein said detained needle seat further comprises an isolating plug therein, said isolating plug being provided at the rear end of junction between said through bore and said shunt tube, said puncture needle passing through said through bore by puncturing said isolating plug.

10. The safe vein detained needle according to Claim 1, wherein said protective seat is provided on the periphery at the front end thereof with a U-shaped stopping portion, a part of tube of said pushing seat is provided with a U-shaped gap, correspondingly, and said curved part of said protective seat is provided at the rear end thereon with a marking curve, wherein said second coupling portion of said withdrawing part being coupled to said first coupling portion of said puncture needle seat when said pushing part is pushed forward such that said gap of said pushing seat is adjoined to said stopping portion, alternatively, said needle head of said puncture needle being returned to be retrieved into said needle head accommodating portion when said pushing part is pushed backward such that said gap of said pushing seat is moved to the top of said marking curve.
